# FASCICULE DE BREVET EUROPEEN

(11) **EP 1 177 815 B1**
(45) Date de publication et mention de la délivrance du brevet: **14.07.2004**
(21) Numéro de dépôt: 01402034.1
(22) Date de dépôt: 27.07.2001
(51) Int. Cl.: A61N 1/375

(54) **Dispositif médical implantable actif tel que stimulateur cardiaque, défibrillateur et/ou cardioverteur comprenant une tête de connecteur démontable**
Aktive implantierbare medizinische Vorrichtung, insbesondere Herzschrittmacher, Defibrillator, und/oder Kardiovertierer, mit einem abnehmbaren Verbindungskopf
Active implantable medical device, in particular pacemaker, defibrillator and/or cardioverter, having a removable connector head

(30) Priorité: 31.07.2000 FR 0010069
(43) Date de publication de la demande: 06.02.2002
(73) Titulaire: ELA MEDICAL, 92541 Montrouge (FR)
(72) Inventeur: Zaouali, Mounir, 75015 Paris (FR); Correas, Philippe, 95230 Soissy Sous Montmorency (FR); D'Hiver, Philippe, 92320 Chatillon (FR)
(74) Mandataire: Dupuis-Latour, Dominique

(56) Documents cités:
- US-A- 4 182 345
- US-A- 5 755 743
- US-A- 5 851 221
- US-A- 5 919 215

## Description

L'invention concerne les connecteurs, notamment pour dispositifs médicaux implantables actifs. Elle sera principalement décrite dans le cas d'un stimulateur cardiaque, mais il ne s'agit là que d'un exemple de mise en oeuvre de l'invention, qui est applicable de façon beaucoup plus générale à une très grande variété de "dispositifs médicaux implantables actifs" tels que définis par la Directive 90/385/CE du 20 juin 1990 du Conseil des communautés européennes, définition qui inclut, outre les stimulateurs cardiaques, les défibrillateurs et/ou cardioverteurs, les appareils neurologiques, les pompes de diffusion de substances médicales, les implants cochléaires, les capteurs biologiques implantés, etc.

Ces dispositifs comportent un générateur contenant l'électronique du dispositif et relié électriquement et mécaniquement à une sonde, le raccordement étant réalisé par le chirurgien au moment de l'implantation.

Plus précisément, ce générateur est constitué d'un boîtier, contenant les divers circuits électroniques et la pile d'alimentation du dispositif, et d'une tête de connecteur reliée mécaniquement et électriquement au boîtier et pourvue d'un ou plusieurs logements susceptibles de recevoir la ou les sonde(s).

Voir par exemple les documents US-A-4 182 345 , US-A-5 919 215 et US-A-5 851 221.

La fabrication du dispositif comporte généralement des étapes consistant, dans un premier temps, à réaliser le boîtier complet, dont émergent sur la face supérieure une pluralité de picots de traversée ; ces picots sont reliés aux circuits internes du boîtier et sont destinés à être reliés chacun à une borne correspondante de la tête de connecteur.

La tête de connecteur est jusqu'à présent généralement montée par assemblage des diverses bornes aux picots correspondants, puis noyage de l'ensemble dans une résine silicone surmoulée assurant à la fois la protection des éléments de la tête, l'étanchéité entre tête et boîtier, et la définition des logements, venus de moulage, destinés à recevoir les extrémités des sondes.

Cette technique de montage simultané de l'ensemble des pièces de la tête et de surmoulage de l'ensemble est une méthode éprouvée et économique, mais elle n'en présente pas moins un certain nombre d'inconvénients, notamment :
- un temps de fabrication important, car il faut attendre le séchage complet de la colle silicone,
- un volume relativement important de la tête de connecteur, en raison du surmoulage qui vient recouvrir toutes les parties de la tête,
- enfin et surtout, en cas de défaut constaté après surmoulage, l'impossibilité de séparer simplement la tête de connecteur et d'isoler la pièce défectueuse pour permettre une reprise de l'assemblage.

Une autre technique consiste à réaliser une tête de connecteur en matériau rigide, par exemple en une résine époxy ou en un dérivé polyuréthanne tel que le *Tecothane*. Le boîtier est pourvu de crochets élastiques en forme d'hameçons coopérant avec des fentes homologues réalisées dans la tête de connecteur, dont la forme permet un déploiement des griffes du crochet à la mise en place de la tête de connecteur.

Cette technique, si elle permet de réduire le temps de fabrication, présente cependant toujours l'inconvénient de l'impossibilité de séparer la tête de connecteur du boîtier une fois la mise en place réalisée.

La présente invention a pour but de proposer une tête de connecteur permettant de remédier à l'ensemble de ces inconvénients, et offrant donc :
- un temps de fabrication réduit, par suppression de l'étape de séchage d'une résine d'enrobage,
- un gain en volume,
- la possibilité de séparer la tête de connecteur du boîtier du dispositif même après montage, ce qui permet en cas de tête défectueuse de récupérer le boîtier pour y monter une autre tête de connecteur,
- la possibilité de contrôler pièce par pièce les différents éléments de structure du connecteur, permettant ainsi, le cas échéant, d'isoler la pièce défectueuse et d'effectuer un travail de reprise sur la tête de connecteur,
- la réduction du nombre de pièces nécessaires pour permettre l'ancrage mécanique de la tête de connecteur.

À cet effet, la présente invention propose un dispositif médical implantable actif tel qu'un stimulateur cardiaque, défibrillateur et/ou cardioverteur, comprenant : un boîtier comportant sur l'une de ses faces une série de picots conducteurs émergeants, reliés aux circuits internes du boîtier ; une tête de connecteur en un matériau isolant rigide, apte à recevoir des bornes de contact électrique ; et un organe de liaison mécanique, apte à solidariser le boîtier à la tête de connecteur, cet organe de liaison étant un organe amovible apte à permettre la séparation de la tête de connecteur d'avec le boîtier après solidarisation de ces derniers.

Selon l'invention, le boîtier comporte au moins un élément apte à coopérer avec l'organe de liaison et formé par une région d'une traversée isolante portant le(s)dit(s) picot(s) conducteur(s).

Très avantageusement, l'élément est de forme homologue de celle de l'organe de liaison au point de contact mutuel, et il s'agit notamment d'une gorge périphérique de la traversée isolante.

Dans un premier mode de réalisation, l'organe de liaison est un organe élastiquement déformable apte à coopérer avec ledit élément homologue du boîtier par encliquetage, notamment une agrafe apte à coopérer par deux extrémités opposées avec au moins deux traversées isolantes du boîtier portant le(s)dit(s) picot(s) conducteur(s).

Dans un deuxième mode de réalisation, l'organe de liaison est un organe rigide apte à coopérer avec ledit élément homologue du boîtier par engagement ajusté, notamment une goupille apte à coopérer avec une traversée isolante du boîtier portant le(s)dit(s) picot(s) conducteur(s).

◇ On va maintenant décrire un exemple de mise en oeuvre de l'invention, en référence aux dessins annexés.
La figure 1 est une vue en perspective, de dessous, d'une tête de connecteur selon l'invention avant mise en place d'une agrafe de solidarisation mécanique.
La figure 2 est identique à la figure 1, après mise en place de l'agrafe.
La figure 3 illustre la manière dont l'agrafe coopère avec les traversées émergeant du boîtier du dispositif.
La figure 4 est une vue isolée de l'une des traversées des figures 1 à 3.
La figure 5 est une variante de la figure 1, où l'agrafe est remplacée par des goupilles de fixation.
La figure 6 illustre, isolément, une traversée adaptée à la variante de la figure 5.

Sur les figures, la référence 10 désigne de façon générale une tête de connecteur selon l'invention, réalisée en un matériau isolant, avantageusement un matériau rigide tel qu'une résine époxy ou un dérivé de polyuréthanne tel que le *Tecothane* (alors que les têtes de connecteur connues sont habituellement réalisées en un matériau souple tel qu'une résine silicone).

Cette tête 10 est destinée à être montée sur le boîtier 12 d'un dispositif médical actif implantable, tel que par exemple un stimulateur cardiaque, ci-après désigné simplement "dispositif'. Ce dispositif est donc constitué d'un corps 12 associé à une tête 10 dans laquelle pourront être introduites les extrémités proximales d'une ou plusieurs sondes, ce raccordement étant réalisé par le chirurgien au moment de l'implantation.

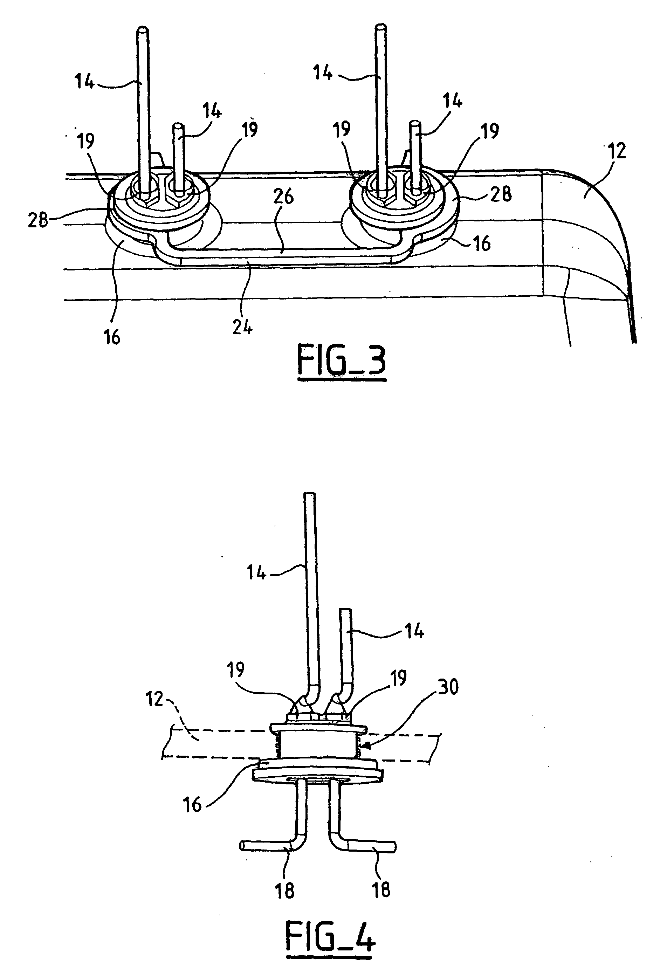

Le corps 12 comporte un boîtier métallique, généralement en titane, contenant l'électronique du dispositif et dont une série de picots 14 (voir notamment figure 3) émergent de l'une des faces via des isolateurs de traversée 16 en un matériau isolant 19 tel qu'une céramique. Ces picots 14 sont des tiges métalliques électriquement reliées aux entrées/sorties du circuit électronique du dispositif par les extrémités opposées 18 soudées à ce circuit, qui est par ailleurs entièrement enfermé dans le corps 12 avec sa pile d'alimentation.

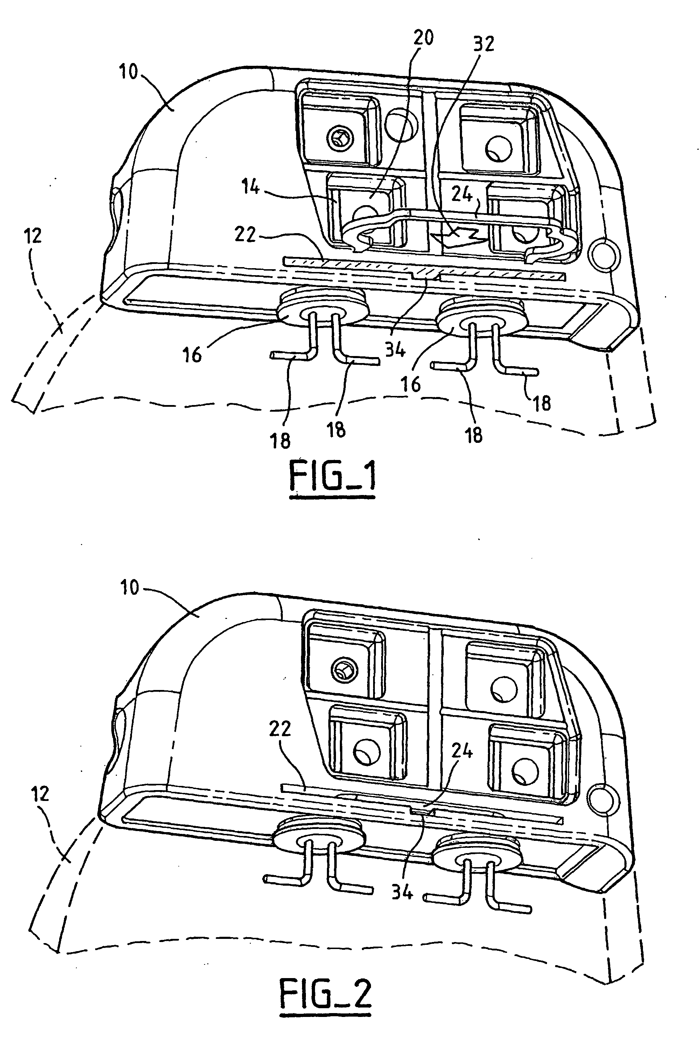

Les picots 14 débouchent (figure 1) dans des cavités correspondantes 20 de la tête de connecteur 10 lorsque cette dernière est montée sur le boîtier 12. Ces cavités 20 logent les bornes de connexion (non représentées) et l'ensemble sera fermé par collage final d'un couvercle de fermeture (non représenté). Les bornes de connexion sont notamment des bornes conformes au standard mécanique dimensionnel dit "IS-1" de la norme française et européenne NF EN 50077 "*Connecteur à bas profil pour stimulateurs cardiaques implantables*", qui définit un système de connexion permettant de garantir l'interchangeabilité des sondes et des générateurs d'impulsions produits par différents fabricants. On soulignera cependant que l'invention n'est pas limitée aux systèmes de connexion selon cette norme, ni même aux systèmes de connexion pour stimulateurs cardiaques.

Dans le mode de mise en oeuvre illustré figures 1 à 4, la tête de connecteur est pourvue à sa base, c'est-à-dire dans sa région proche du boîtier 12, d'une fente longitudinale 22 s'étendant dans un plan parallèle à la face supérieure du boîtier 12. Cette fente 22 est homologue d'une agrafe élastique 24, fente et agrafe étant dimensionnées de manière que l'agrafe 24 puisse être entièrement introduite dans la fente 22, comme illustré figure 2.

L'agrafe 24, comme on peut le voir plus précisément figure 3, est constituée d'une partie centrale rectiligne 26, et de deux parties d'extrémité semblables 28 de forme arrondie destinées à venir s'encliqueter dans une gorge homologue 30 (figure 4) de la traversée 16.

Dans la réalisation illustrée, la section de l'agrafe 24 est rectangulaire, correspondant à une forme complémentaire de la gorge 30 de la traversée 16.

L'agrafe est avantageusement réalisée en alliage de titane ou en acier inoxydable, et ses dimensions sont choisies en fonction de la forme et du positionnement respectif des traversées 16 de manière qu'elle puisse s'encliqueter à cheval sur les deux traversées 16 par déformation élastique, de manière réversible, par simple enfoncement manuel sans nécessité d'outillage particulier.

Le processus de montage de la tête de connecteur sur le boîtier est le suivant.

Le corps du dispositif a été déjà assemblé, et se présente sous la forme d'un boîtier métallique d'où émergent les deux traversées 16 portant les picots 14.

La tête de connecteur 10 est alors posée sur la face correspondante du boîtier, et l'opérateur vient insérer (flèche 32, figure 1) et encliqueter l'agrafe 24 autour des deux traversées 16, assurant par cette seule opération la solidarisation mécanique de la tête 10 au corps 12. L'ensemble boîtier-tête résultant se présente comme illustré figure 2.

L'ensemble formé par la tête 10 et les bornes destinées à être reliées à chacun des picots émergeant dans les cavités 20 (bornes femelles du système de connexion) est assemblé par soudage de chacune des extrémités des picots 14 à sa borne respective.

En variante, il est possible de réaliser cette opération de soudure avant solidarisation mécanique de la tête au connecteur, mais dans ce cas un outillage de positionnement et de maintien de la tête sur le corps est nécessaire.

L'étape finale est une étape de collage, à savoir collage d'un couvercle de fermeture des cavités 20, et collage final de la tête de connecteur au boîtier avec enrobage de ce dernier, pour assurer une liaison définitive entre ces deux éléments.

Si, avant l'étape de collage final, l'opérateur repère un défaut, notamment une soudure défectueuse, il est possible - de façon caractéristique de l'invention - de désolidariser la tête du boîtier afin de recommencer l'opération, ou d'échanger la tête contre une autre.

Plus précisément, la désolidarisation est effectuée par extraction de l'agrafe 24, simplement en exerçant au moyen d'une pince une traction dans la partie centrale de cette agrafe. Une encoche 34 (figure 2) permet l'introduction aisée des becs de la pince pour saisir l'agrafe 24. Les picots de traversée sont découpés au niveau de la soudure, et la tête est retirée. Une deuxième soudure du même picot sur une nouvelle borne sera possible, dès lors que sont prévues une longueur de picot suffisante et une surface de soudure sur les bornes suffisante pour pouvoir refaire cette deuxième soudure.

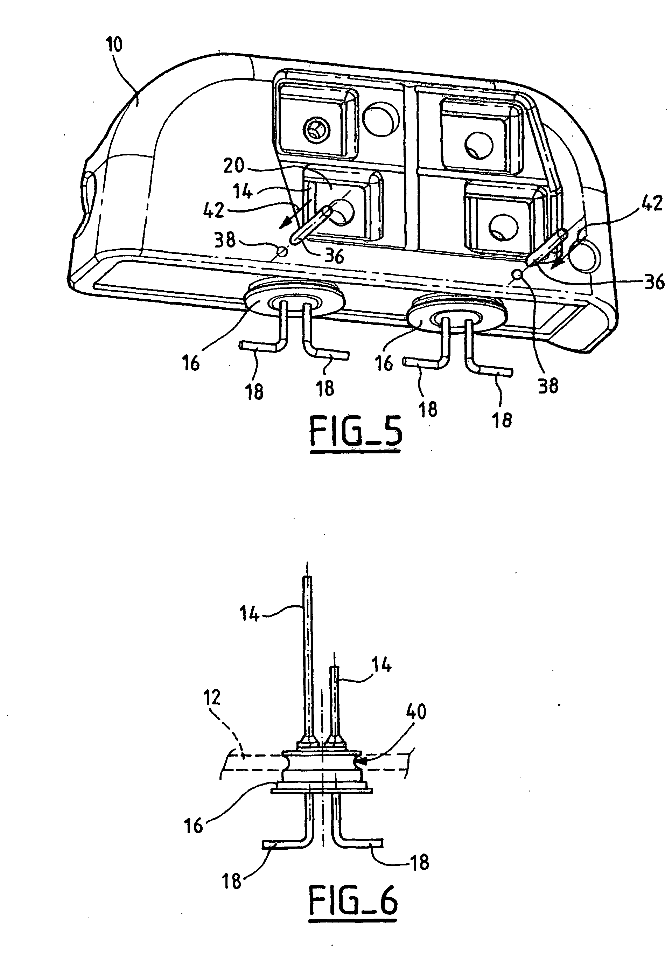

Les figures 5 et 6 illustrent une variante de réalisation de l'invention, où l'agrafe 24 est remplacée par des goupilles 36 insérées dans des trous homologues 38 de la tête de connecteur 10. Les trous 38 débouchent au droit d'une gorge 40 formée dans la face latérale de la traversée 16. Cette gorge 40 est formée, comme dans le cas de la gorge 30 de la première variante, par une surface de révolution de profil complémentaire de celui de l'organe de fixation : ici, un profil circulaire concave dont le rayon correspond à celui des goupilles cylindriques 36.

Dans cette deuxième variante, la solidarisation de la tête de connecteur au boîtier se fait par enfoncement (flèches 42) des goupilles 36 dans les trous 38.

La séparation de la tête est effectuée par extraction des goupilles, en les poussant par un orifice situé en vis-à-vis des trous 38 et de plus faible diamètre que ceux-ci (les trous 38 étant des trous débouchants).

On notera que, dans cette variante de réalisation aussi bien que dans la première, la fixation mécanique de la tête au corps ne nécessite pas de pièce complémentaire sur le boîtier : l'organe de fixation (agrafe 24 ou goupilles 36) s'engage en effet directement sur une gorge homologue du corps de la traversée 16.

Cette fonction additionnelle du corps de traversée (fonction de contribution à la solidarisation mécanique) est très économique, puisqu'elle permet de réduire le nombre de pièces et les temps d'assemblage intermédiaires, pour un surcoût négligeable sur le corps de la traversée, la gorge 30 ou 40 étant une simple forme de révolution usinée dans le corps de la traversée 16.

## Revendications

1. Un dispositif médical implantable actif tel qu'un stimulateur cardiaque, défibrillateur et/ou cardioverteur, comprenant :
- un boîtier (12) comportant sur l'une de ses faces une série de picots conducteurs émergeants (14), reliés aux circuits internes du boîtier,
- une tête de connecteur (10) en un matériau isolant rigide, apte à recevoir des bornes de contact électrique, et
- un organe de liaison mécanique (24 ; 36, 36), apte à solidariser le boîtier à la tête de connecteur, cet organe de liaison étant un organe amovible apte à permettre la séparation de la tête de connecteur d'avec le boîtier après solidarisation de ces derniers,
**caractérisé en ce que** le boîtier comporte au moins un élément (30 ; 40) apte à coopérer avec l'organe de liaison (24 ; 36, 36) et formé par une région d'une traversée isolante (16) portant le(s)dit(s) picot(s) conducteur(s).

2. Le dispositif de la revendication 1, dans lequel ledit élément est de forme homologue de celle de l'organe de liaison au point de contact mutuel.

3. Le dispositif de la revendication 1, dans lequel ladite région est une gorge périphérique (30 ; 40) de la traversée isolante.

4. Le dispositif de la revendication 2, dans lequel l'organe de liaison est un organe élastiquement déformable apte à coopérer avec ledit élément homologue du boîtier par encliquetage.

5. Le dispositif de la revendication 4, dans lequel l'organe élastiquement déformable est une agrafe (24) apte à coopérer par deux extrémités opposées (28, 28) avec au moins deux traversées isolantes du boîtier portant le(s)dit(s) picot(s) conducteur(s).

6. Le dispositif de la revendication 1, dans lequel l'organe de liaison est un organe rigide apte à coopérer avec ledit élément homologue du boîtier par engagement ajusté.

7. Le dispositif de la revendication 6, dans lequel l'organe rigide est une goupille (36) apte à coopérer avec une traversée isolante du boîtier portant le(s)dit(s) picot(s) conducteur(s).

## Patentansprüche

1. Aktive implantierbare medizinische Vorrichtung, wie ein Herzschrittmacher, Defibrillator und/oder Kardioverter, umfassend:
- ein Gehäuse (12), auf einer seiner Seiten eine Reihe von hervorragenden leitenden Stiften (14) aufweisend, verbunden mit den internen Schaltungen des Gehäuses,
- einen Steckerkopf (10) aus starrem isolierendem Material, geeignet, elektrische Kontaktanschlüsse zu empfangen, und
- ein mechanisches Verbindungsmittel (24; 36, 36), geeignet, das Gehäuse an dem Kopf des Steckers zu befestigen, wobei Verbindungsmittel ein unbewegliches Mittel ist, geeignet, die Trennung des Steckerkopfs von dem Gehäuse zu erlauben, nach Verbindung dieser beiden,
**dadurch gekennzeichnet, dass** das Gehäuse mindestens ein Element (30; 40) aufweist, geeignet, mit dem Verbindungsmittel (24; 36, 36) zusammenzuwirken, und gebildet durch einen Bereich einer isolierenden Durchführung (16), welche den/die leitenden Stift(e) trägt.

2. Vorrichtung gemäß Anspruch 1, in welcher das Element eine Form besitzt, die homolog zu derjenigen des Verbindungsmittels ist, am gegenseitigen Kontaktpunkt.

3. Vorrichtung gemäß Anspruch 1, in welcher der genannte Bereich eine umlaufende Rille (30; 40) der isolierenden Durchführung ist.

4. Vorrichtung gemäß Anspruch 2, in welcher das Verbindungsmittel ein elastisch verformbares Mittel ist, geeignet, mit dem genannten homologen Element des Gehäuses durch Einrasten zusammenzuwirken.

5. Vorrichtung gemäß Anspruch 4, in welcher das elastisch verformbare Mittel ein Bügel (24) ist, geeignet, durch zwei gegenüberliegende Enden (28, 28) mit mindestens zwei isolierenden Durchführungen des Gehäuses zusammenzuwirken, welche den/die leitenden Stift(e) trägt.

6. Vorrichtung gemäß Anspruch 1, in welcher das Verbindungsmittel ein starres Mittel ist, geeignet, mit dem homologen Element des Gehäuses durch angepassten Formschluss zusammenzuwirken.

7. Vorrichtung gemäß Anspruch 6, in welchem das starre Mittel ein Stift (36) ist, geeignet, mit einer isolierenden Durchführung des Gehäuses zusammenzuwirken, welche den/die leitenden Stift(e) trägt.

## Claims

1. An active implantable medical device such as pacemaker, defibrillator and/or cardioverter, comprising:
- a case (12) comprising on one of its surfaces a series of protruding conducting leads (14), connected to the internal circuits of the case,
- a connector head (10) made of a rigid insulating material, adapted to receive electrical contact terminals, and
- a mechanical connection member (24; 36, 36), adapted to fasten the case to the connector head, said connection member being a removable member adapted to enable the separation of the connector head from the case after the latter having been fastened together,
**characterised in that** the case includes at least one element (30; 40) adapted to cooperate with the connection member (24; 36, 36) and made from an area of an insulating feed-through (16) supporting said conductive lead(s).

2. The device of claim 1, wherein said element has a shape which matches the shape of the connection member at the point of mutual engagement.

3. The device of claim 1, wherein said area is a peripheral groove (30; 40) of the insulating feed-through.

4. The device of claim 2, wherein the connection member is an elastically deformable member adapted to cooperate with said matching element by snap-on engagement.

5. The device of claim 4, wherein the elastically deformable component is a clamp (24) adapted to cooperate by means of two opposite ends (28, 28) with at least two insulating feed-throughs of the case supporting said conductive lead(s).

6. The device of claim 1, wherein the connection member is a rigid member adapted to cooperate with said matching element of the case by snug fit engagement.

7. The device of claim 6, wherein the rigid member is a pin (36) adapted to cooperate with an insulating feed-through of the case supporting said conductive lead(s).
